# EUROPEAN PATENT SPECIFICATION

(11) **EP 3 079 620 B1**
(45) Date of publication and mention of the grant of the patent: **21.11.2018**
(21) Application number: 14815550.0
(22) Date of filing: 03.12.2014
(51) Int. Cl.: A61B 18/14

(54) **BIPOLAR ELECTROSURGICAL DEVICE WITH A RECESSED DISTAL PORTION FOR A RETURN ELECTRODE**
BIPOLARE ELEKTROCHIRURGISCHE VORRICHTUNG MIT AUSGESPARTEM DISTALEM BEREICH FÜR EINE NEUTRALELEKTRODE
DISPOSITIF ÉLECTRO-CHIRURGICAL BIPOLAIRE À PARTIE DISTALE AVEC UNE NICHE POUR UNE ÉLECTRODE DE RETOUR

(30) Priority: 05.12.2013 US 201361912325 P
(43) Date of publication of application: 19.10.2016
(73) Proprietor: Cook Medical Technologies LLC, Bloomington, IN 47404 (US)
(72) Inventor: RIVERA, Stephanie, Burlington, NC 27215 (US); GAYZIK, Caroline, Winston-salem, NC 27106 (US); RUCKER, Laura, Greensboro, NC 27406 (US); ABDELHAQ, Shaq, Greensboro, NC 27410 (US)
(74) Representative: Garratt, Peter Douglas
(86) International application number: PCT/US2014/068272
(87) International publication number: WO 2015/084907

(56) References cited:
- EP-A1- 0 959 786
- EP-A2- 0 440 385
- US-A1- 2012 310 265

## Description

### TECHNICAL FIELD

The present invention relates generally to medical devices, and more particularly to bipolar sphincterotomes having a return electrode disposed in a recess at a distal portion of the bipolar sphincterotome.

### BACKGROUND

A sphincterotome is a medical device that is used to perform a sphincterotomy, which involves cutting a sphincter muscle, such as the sphincter of Oddi. The sphincter muscle may need to be cut to relieve its constrictive nature and allow one or more medical devices through the muscle. For example, problems occurring in the biliary tree, such as the formation of bile duct stones or papillary stenosis, may be treated using medical devices that are delivered into the biliary tree. In order to access the biliary tree, the medical devices may pass through the sphincter of Oddi. To facilitate passage of the medical devices through the sphincter of Oddi, the sphincter muscle may be cut using a sphincterotome.

A sphincterotome may generally include an elongate tubular member, such as a catheter, and a cutting wire that is used to cut the sphincter muscle. The cutting wire may extend through a lumen of the catheter, except at a distal portion of the catheter, where the cutting wire may project from and be exposed outside of the catheter. The exposed portion, which may be referred to as a cutting edge, may be used to cut the sphincter muscle.

A sphincterotomy generally involves a two-part process: cannulation of the biliary tree and cutting the sphincter muscle by sending electric current through the cutting wire (i.e, electrosurgery). Cannulation of the biliary tree may include inserting the distal portion of the catheter into the papilla and using the distal portion and the cutting edge to lift an upper portion (i.e., the roof) of the papilla. The roof of the papilla may be lifted by proximally pulling the cutting wire taut, causing the distal portion of the tubular member to bow and form an arc. After cannulation, the electric current may be provided to the cutting edge to cut the sphincter muscle.

Reference is directed to EP 0959786 which discloses an electrosurgical instrument, for the treatment of tissue in the presence of an electrically-conductive fluid medium, comprises an instrument shaft, and an electrode assembly at one end of the shaft. The electrode assembly comprises a tissue treatment electrode and a return electrode which is electrically insulated from the tissue treatment electrode by means of an insulation member. The tissue treatment electrode has an exposed end extending laterally through a cut-out provided in the insulation member at the distal end portion of the instrument. The return electrode has a fluid contact surface which overlies the insulation member in the region of the cut-out. The fluid contact surface is spaced from the tissue treatment electrode in such a manner as to define, in use, a conductive fluid path that completes an electrical circuit between the tissue treatment electrode and the return electrode.

Reference is further directed to EP 0440385 which discloses a bipolar sphinctertome for use in endoscopic retrograde sphincterotomy procedures including an elongated, flexible, plastic tube having either a single or a double lumen and including a segment near its distal end which is both flexible and conductive. This segment is connected by a wire extending through the lumen to a terminal at the proximal end of the device adapted to be coupled to an electrosurgical generator. A second, longitudinally movable wire passes through the lumen and is connected at the proximal end to a finger-operated slide mechanism while its distal end passes through a small aperture formed in the tube just proximal of the flexible conductive segment. That wire is then anchored distally of the flexible conductive segment so that when a tensioning force is applied to the wire, the distal end portion of the instrument will bow, with the exposed wire comprising the active electrode and the flexible conductive segment functioning as the return electrode. A two-part handle assembly permits the sphinctertome to be treated as a disposable, single-use surgical instrument.

### BRIEF SUMMARY

In a first aspect, a bipolar electrosurgical device may be configured to perform an electrosurgical procedure at a treatment site within a patient. The electrosurgical device may include an elongate tubular member longitudinally extending from a proximal portion to a distal portion. The tubular member may include: a body; an active wire lumen longitudinally extending in the body; and a recess extending in the body at the distal portion. The electrosurgical device may further include an active path configured for delivery of electrical current generated from a power source to the distal portion, wherein the active path comprises an exposed active portion disposed outside of the tubular member to contact tissue at the treatment site; and a return path for return of the electrical current from the distal portion to the power source. The return path comprises a return electrode disposed in the recess at the distal portion.

In a second aspect, a bipolar electrosurgical device may include an elongate tubular member comprising: a body; an active path lumen longitudinally extending in the body; and a return path lumen longitudinally extending in the body. The bipolar electrosurgical device may also include a conductive active path comprising an active wire longitudinally extending in the active path lumen; and an exposed active portion coupled to the active wire and disposed outside of the tubular member. The bipolar electrosurgical device may further include a conductive return path longitudinally extending in the return path lumen; and a gap extending in the body at a distal portion of the tubular member. The gap may expose a return electrode portion of the return path to outer surroundings of the tubular member.

### BRIEF DESCRIPTION OF THE DRAWINGS

FIG. 1 is a side view of an example bipolar sphincterotome electrically connected to a power source.
FIG. 1A is a side view of the distal portion of the bipolar sphincterotome of FIG. 1, with an alternative configuration of a return electrode.
FIG. 1B is a side view of a distal portion of the bipolar sphincterotome of FIG. 1, with another alternative configuration of the return electrode.
FIG. 2 is a cross-sectional axial view of the bipolar sphincterotome of FIG. 1, showing a circumferential positioning of a recess and the return electrode.
FIG. 3 is a cross-sectional axial view of the bipolar sphincterotome of FIG. 1, showing an alternative circumferential positioning of the recess and the return electrode.
FIG. 4 is a perspective view of the distal portion of the bipolar sphincterotome of FIG. 1, where the return electrode is configured as a single continuous piece of conductive material.
FIG. 5 is a perspective view of the distal portion of the bipolar sphincterotome of FIG. 1, where the return electrode is configured as multiple pieces of conductive material.
FIG. 6 is a perspective view of the distal portion of the bipolar sphincterotome of FIG. 1, where the return electrode includes a plurality of wires longitudinally extending in the recess.
FIG. 7 is a perspective view of the distal portion of the bipolar sphincterotome of FIG. 1, where the return electrode is return electrode includes a plurality of configured as a mesh or in a braided configuration.
FIG. 8 is a perspective view of the distal portion of the bipolar sphincterotome of FIG. 1, where the return electrode is configured as a plurality of wires angled relative to a longitudinal axis.
FIG. 9 is a cross-sectional side view of an example configuration of a tubular member of the bipolar of sphincterotome of FIG. 1, where the sphincterotome if shown in isolation.
FIG. 10 is a cross-sectional axial view of the tubular member shown in FIG. 9.
FIG. 11 is a cross-sectional side view of the tubular member shown in FIG. 9 integrated with a return path.
FIG. 11A is a cross-sectional axial view of the tubular member and the return path shown in FIG. 11.
FIG. 12 is a cross-sectional side view of another example configuration of the tubular member of the bipolar sphincterotome of FIG. 1, where the tubular member has multiple return lumens.
FIG. 13 is a cross-sectional axial view of the tubular member shown in FIG. 12, with conductive material disposed in the return lumens.
FIG. 14 is another cross-sectional axial view of the tubular member shown in FIG. 12, showing a pair of return electrode portions being separated by a divider extending in the recess.
FIG. 15 is a cross-sectional side view of another example configuration of the tubular member of the bipolar sphincterotome of FIG. 1, where the tubular member has multiple return lumens.
FIG. 16 is a cross-sectional axial view of the tubular member shown in FIG. 15, with conductive material disposed in the return lumens.
FIG. 17 is another cross-sectional axial view of the tubular member shown in FIG. 15, where a divider does not extend in the recess and the return electrode is a single continuous piece of conductive material.
FIG. 18 is cross-sectional side view of the tubular member, where the return path includes a coil embedded in the body.

### DETAILED DESCRIPTION

The present disclosure describes bipolar electrosurgical devices that include an elongate tubular member, a recess disposed in a distal portion of the tubular member, and a return electrode disposed in the recess. For some example configurations, the recess may be formed at least in part by a lumen extending in the tubular member. In the recess, the return electrode may be exposed to outside the tubular member to contact tissue at a treatment site within a patient for performance of an electrosurgical procedure. The return electrode disposed in the recess may be an alternative to configurations where the return electrode is disposed on or about an outer surface of the elongate tubular member.

The bipolar electrosurgical device (also referred to as an electrosurgical device having a bipolar configuration) may be any electrosurgical device configured to perform an electrosurgical procedure in a bipolar manner. The bipolar electrosurgical device may include an active path and a return path that may be electrically coupled to a power source. The active path may longitudinally extend within the elongate tubular member and may supply electrical current generated by the power source to the treatment site. The return path may return the supplied current back to the power source. By having a bipolar configuration, the return path may be attached to, adhered to, integrated with, disposed within, or included as part of the elongate tubular member.

The active path may include an exposed active portion that contacts and supplies current to tissue at the treatment site. For some bipolar electrosurgical devices, where the exposed portion of the active path radially extends outward from the tubular member, the return electrode disposed in the recess may be circumferentially offset a predetermined number of degrees from the radial positioning of the exposed active portion. Alternatively, the return electrode may not be circumferentially offset from the exposed portion of the active path, such as where the exposed active portion distally extends beyond a distal end of the tubular member to be exposed for contact with the tissue.

Example bipolar electrosurgical device may include bipolar sphincterotomes, needle knives, snares, or forceps, as examples. Other bipolar electrosurgical devices may be possible. The following description describes a return electrode disposed in a recess for various example embodiments of a bipolar sphincterotome that is configured to perform a sphincterotomy at a treatment site within a patient. However, the features described are not limited to bipolar sphincterotomes and may be applied or implemented with other bipolar electrosurgical devices.

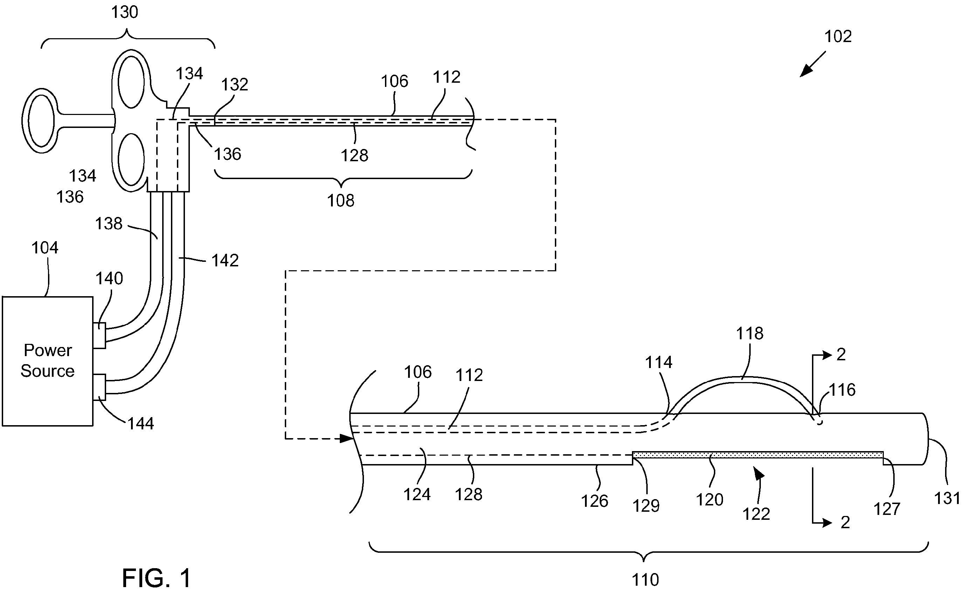

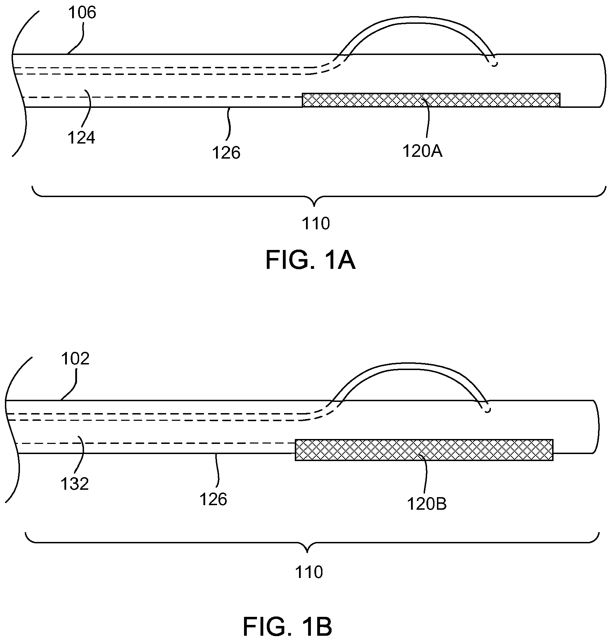

Fig. 1 shows a side view of an example bipolar sphincterotome 102 in electrical connection with a power source 104. The power source 104 may be an electronic device, such as a radio frequency (RF) generator or an electrosurgical unit (ESU) that is configured to generate and supply electrical current to the bipolar sphincterotome 102 for performance of the sphincterotomy. The bipolar sphincterotome 102 may include an elongate, tubular member 106 that longitudinally extends from a proximal portion 108 to a distal portion 110. The bipolar sphincterotome 102 may also include an active path and a return path that may be electrically coupled to the power source 104. The active path may be configured to deliver electrical current from the power source 104 to the treatment site, and the return path may return the electrical current back to the power source 104.

The active path may include a cutting wire 112 that is used to cut the sphincter muscle. The cutting wire 112 may longitudinally extend within a cutting wire lumen (not shown in Fig. 1) from the proximal portion 108 to the distal portion 110. At the distal portion 110, the cutting wire 112 may extend or protrude from within the tubular member 106, through a first opening 114 of the tubular member 106, to outside the tubular member 106. Outside the tubular member 106, an exposed portion 118 of the cutting wire 112 may longitudinally extend substantially parallel with the tubular member 106 to a second opening or anchor point 116 of the tubular member 106 that is distal to the first opening 114, where a distal end of the cutting wire 112 may re-enter and/or be fixedly attached to the tubular member 106. The exposed portion 118 of the cutting wire 112 may be referred to as a cutting edge, which may be the portion of the cutting wire 112 that cuts tissue (e.g., the sphincter muscle) at the treatment site.

In accordance with a bipolar configuration, the return path of the bipolar sphincterotome 102 may be attached to, adhered to, integrated with, disposed within, or included as part of the tubular member 106. The bipolar configuration may differ from a monopolar configuration, which may use a neutral electrode (e.g., a solid, neutral electrode or a split neutral electrode) positioned on a thigh of the patient as part of the return path.

The return path may include a return electrode 120 disposed at the distal portion 110 of the tubular member 106. The return electrode 120 may be a portion of the return path that is configured to contact tissue at the treatment site. Accordingly, the return electrode 120 may be a conductive, exposed portion of the return path that is exposed to the outer surroundings of the bipolar sphincterotome 102 at the distal portion 110 so that the return electrode 120 may contact the tissue.

As shown in Fig. 1, the return electrode 120 may be disposed in a recess or recessed portion 122 at the distal portion 110 of the tubular member 106. The recess 122 may be a space or gap extending in a body 124 of the tubular member 106 from an outer surface 126 to within the body 124. Longitudinally, the recess 122 and the return electrode 120 may extend to a distal position 127 that is distally past the anchor point 116. Extending the recess 122 and the return electrode 120 distally past the anchor point 116 may ensure or increase the likelihood that the return electrode 120 sufficiently contacts the sphincter muscle or tissue near the sphincter muscle at the treatment site. The distal position 127 may be located before a distal tip 131 and/or sufficiently away from an opening of a wire guide lumen (not shown in Fig. 1) at the distal tip 131 so that a wire guide in the wire guide lumen is not part of (i.e., is electrically isolated) from the return path. In addition, the recess 122 and the return electrode 120 may proximally extend to a proximal position 129 that is located proximal to the cutting wire first opening 114.

In addition, as shown in Fig. 1, when disposed in the recess 122, the return electrode 120 may be recessed from the outer surface 126. In alternative configurations, the return electrode 120 may be flush or substantially flush with the outer surface 126, as shown in by return electrode 120A in Fig. 1A, or may protrude from the outer surface 126, as shown by return electrode 120B in Fig. 1B.

Referring back to Fig. 1, the return path may also include a conductive, unexposed member 128 electrically connected to the return electrode 120. The unexposed member 128 may be disposed distal the return electrode 120 and configured to deliver electrical current from the return electrode 120 back to the power source 104. The unexposed member 128 may be unexposed to the outer surroundings of the bipolar sphincterotome 102 and/or be the portion of the return path that is configured to not contact tissue within the patient. In the example configuration shown in Fig. 1, the unexposed member 128 may longitudinally extend within the tubular member 106 from the proximal portion 108 to the distal portion 110, where the unexposed member 128 is electrically connected to the exposed, return electrode 120. In alternative example configurations, all or some of the unexposed member 128 may longitudinally extend outside of the tubular member 106, such as alongside the outer surface 126. For these configurations, the unexposed member 128 may be encased or coated with an insulating material so that the conductive material of the unexposed member 128 does not contact the tissue.

The bipolar sphincterotome 102 may further include a handle assembly 130 coupled to a proximal end 132 of the tubular member 106. The handle assembly 130 may be operatively coupled to a proximal end 134 of the cutting wire 112, and configured to proximally and distally move the cutting wire 112 to position the distal portion 110 of the tubular member in taut and relaxed positions during use of the bipolar sphincterotome 102. In addition, as shown in Fig. 1, the proximal end 134 of the cutting wire 112 and a proximal end 136 of the unexposed member 128 may each be electrically coupled to the power source 104 via the handle assembly 130, although alternative configurations may be possible. An active power cord 138 may be connected to an active port 140 of the power source 104 and to the handle assembly 130 to electrically couple the active port 130 of the power source 104 with the active wire 112. Similarly, a return power cord 142 may be connected to a return port 144 of the power source 104 and to the handle assembly 130 to electrically couple the unexposed member 128 of the return path to the return portion 144 of the power source 104.

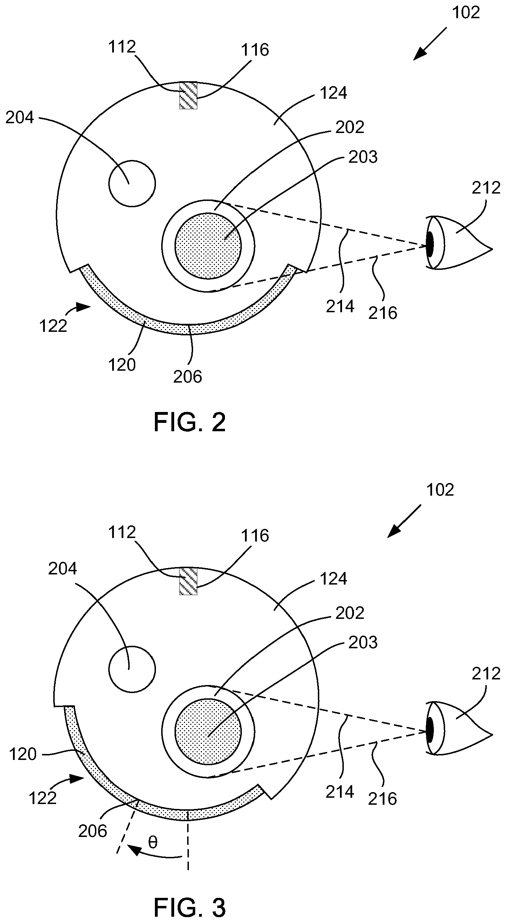

Fig. 2 shows a cross-sectional axial view of an example configuration of the bipolar sphincterotome 102 taken along line 2-2 in Fig. 1. The tubular member 106 may include a wire guide lumen 202 that longitudinally extends in the body 124. The wire guide lumen 202 may be configured to receive and have movably disposed therethrough a wire guide 203. In operation, the wire guide 203 may be delivered to the treatment site within the patient. The wire guide lumen 202 may be inserted over the wire guide 203, and the distal portion 110 of the bipolar sphincterotome 102 may be delivered to the treatment site. In addition, the tubular member 102 may include one or more other lumens longitudinally extending in the body 124, such as an injection lumen 204, which may be used to deliver contrast to the treatment site.

The recess 122 and the return electrode 120 disposed in the recess 122 may circumferentially extend about the tubular member 106. As shown in Fig. 2, the recess 122 and the return electrode 120 may circumferentially extend lengths that are less than a total circumference or circumferential length of the tubular member 106. For some example configurations, as shown in Fig. 2, the recess 122 and the return electrode 120 may circumferentially extend less than half the total circumference of the tubular member 106. For alternative configurations, the recess 122 and the return electrode 120 may circumferentially extend half or more than half the total circumference. In general, the recess 122 and the return electrode 120 may circumferentially extend a sufficient length to ensure that the return electrode sufficiently contacts tissue at the treatment site in accordance with a desired and/or predetermined contact current density ratio between the return electrode 120 and the cutting edge 118.

In addition, from a circumferential perspective, the recess 122 and the return electrode 120 may be circumferentially positioned in the tubular member 106 relative to a circumferential position of the cutting edge 118 and/or the second opening or anchor point 116, where the cutting edge 118 may be distally anchored to the tubular member 106. For the example configuration shown in Fig. 2, a midpoint 206 of the return electrode 120 and/or the recess 122 may be circumferentially positioned to be opposite the circumferential position of the cutting edge 118 and/or the anchor point 116 or offset about 180 degrees about a central longitudinal axis of the tubular member 106 from where the anchor point 116 is positioned and/or where the cutting edge 118 radially extends from the central longitudinal axis. Alternatively, the circumferential position of the midpoint 206 may be offset a predetermined number of degrees 0 about the central longitudinal axis from the 180-degree position, as shown in Fig. 3.

For some situations, the offset positioning shown in Fig. 3 may be preferred over the 180-degree positioning shown in Fig. 2 where an opaque material of the return electrode 120 in conjunction with its 180-positioning impairs visual access by a camera system, represented by eyeball 212, to the wire guide lumen 202 within the tubular member 106. That is, the tubular member 106 may be made of a transparent or semi-transparent material that provides visual access to the wire guide lumen 202 within the tubular member 106. Where the 180-degree positioning of the recess 122 and return electrode 120 at least partially impairs visual access to the wire guide lumen 202, as shown by dotted lines 214, 216 in Fig. 2, the midpoint 206 the return electrode 120 and recess 122 may be circumferentially rotated a predetermined number of degrees 0 from the 180-degree positioning to remove any visual impairment, as shown in Fig. 3.

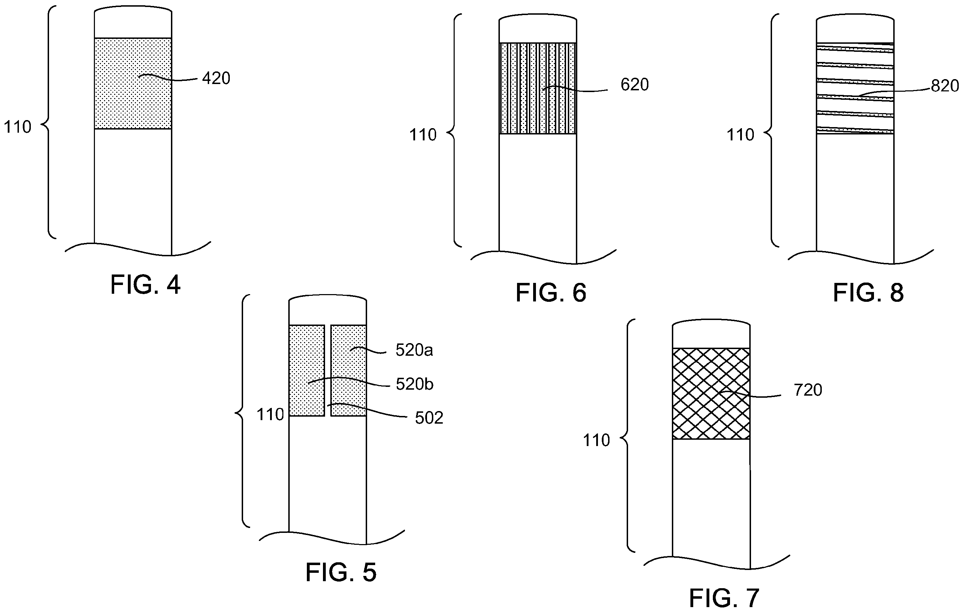

Referring to Figs. 4-7, the return electrode 120 may be made of various conductive materials, structure, and/or have various configurations. In one example configuration shown in Fig. 4, the return electrode may be a single solid and/or continuous piece of conductive material 420. The single, solid piece of conductive material may be a flat or substantially flat conductive sheet of metal, conductive film, conductive ink, conductive paste, or some combination thereof.

In another example configuration shown in Fig. 5, the return electrode may be made of a plurality, such as a pair, of return electrode portions 520a, 520b, each portion 520a, 520b being a single solid piece of conductive material, such as a conductive sheet, conductive film, conductive ink, or conductive paste, as examples. The return electrode portions 520a, 520b may be separated or spaced apart from each other by a spacer or gap 502. Fig. 5 shows the spacer 502 having a generally straight shape that longitudinally extends between the pair of return electrode portions 520a, 520b. In alternative configurations, the spacer 502 may have a non-straight pattern, such as a zig-zag or sinusoidal pattern as examples.

In other example configurations shown in Figs. 6-8, the return electrode may include one or more conductive wires. The wires may be flat wires (e.g., wires having a flat or substantially flat axial cross-section), or circular wires (e.g., wires having a circular or substantially circular axial cross-section). Additionally, the wires may be made of various conductive materials. For example, the wires may be made of a shape memory alloy such as nitinol. Alternatively, the wires may be made of stainless steel or tungsten. Other conductive materials may be used. In the example configuration shown in Fig. 6, the one or more wires may include a plurality of wires 620 longitudinally or substantially longitudinally extending in the recess 122. In another example configuration shown in Fig. 7, the one or more wires may include a plurality of wires 720 linked together as a mesh or in a braided or other type of weaved configuration. In another example configuration shown in Fig. 8, the one or more wires 820 may extend in the recess 122 in a direction that is generally transverse or angled relative to the longitudinal direction that the body 124 extends.

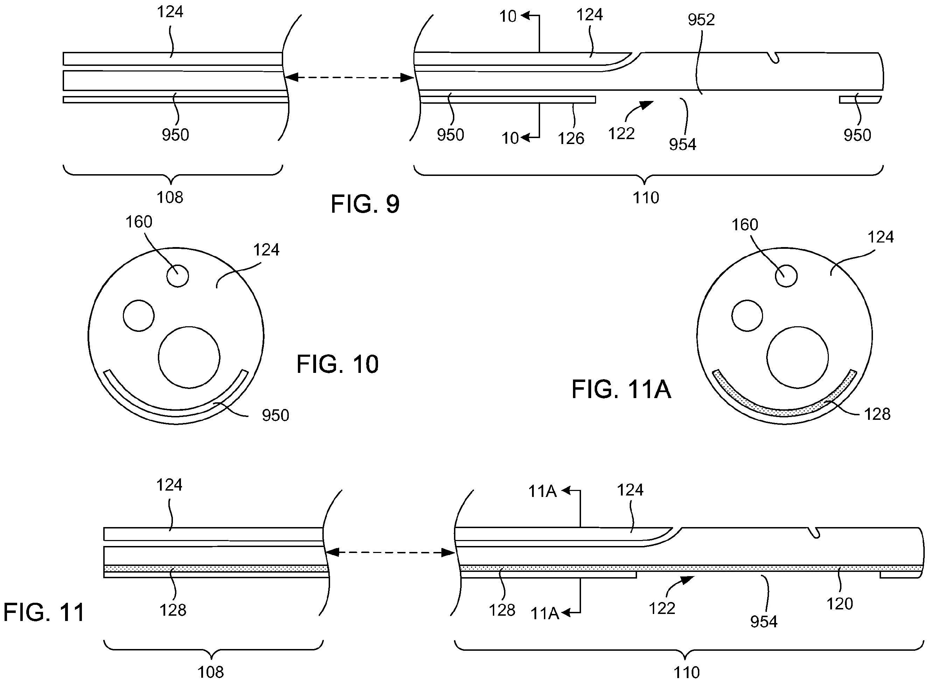

Fig. 9 shows a cross-sectional side view of an example configuration of the tubular member 106 in isolation. In the example configuration, the tubular member 106 may include a return lumen 950 longitudinally extending in the body 124 from the proximal portion 108 to the distal portion 110. For some example configurations, the return lumen 902 may have an axial cross-section that is arc-shaped, as shown in the cross-sectional view of Fig. 10 taken along line 10-10 in Fig. 9. Axial cross-sectional shapes other than an arc may be used for the return lumen 902.

The recess 122 at the distal portion 110 may include and/or be formed in part by a recess portion 952 of the return lumen 950. The recess 122 may further include and/or be formed by a spacing or gap 954 in the body 124 that inwardly extends from the outer surface 126 to the recess portion 952 of the return lumen 950. The spacing or gap 954 may expose the recess portion 952 of the return lumen 950 to the outer surroundings of the tubular member 106. The return electrode 120 and the unexposed member 128 of the return path may then be disposed in the return lumen 950, as shown in Figs. 11 and 11A.

During manufacture, the return lumen 950 may be formed in the body 124 by an extrusion process. After extruding the return lumen 950, conductive material making up the return electrode 120 and the unexposed member 128 of the return path may be inserted into the return lumen 950, as shown in Figs. 11 and 11A. Additionally, the spacing or gap 954 may be formed by removing or detaching a corresponding portion of the body 124. In some example methods of manufacture, the conductive material may be inserted in the return lumen 950 before forming the spacing or gap 954. Accordingly, removing a portion of the body 124 to form the spacing or gap 954 may in turn expose the portion of the conductive material making up the return electrode 120 to the outer surroundings of the tubular member 106. Alternatively, the conductive material may be inserted in the return lumen 950 after forming the spacing or gap 954.

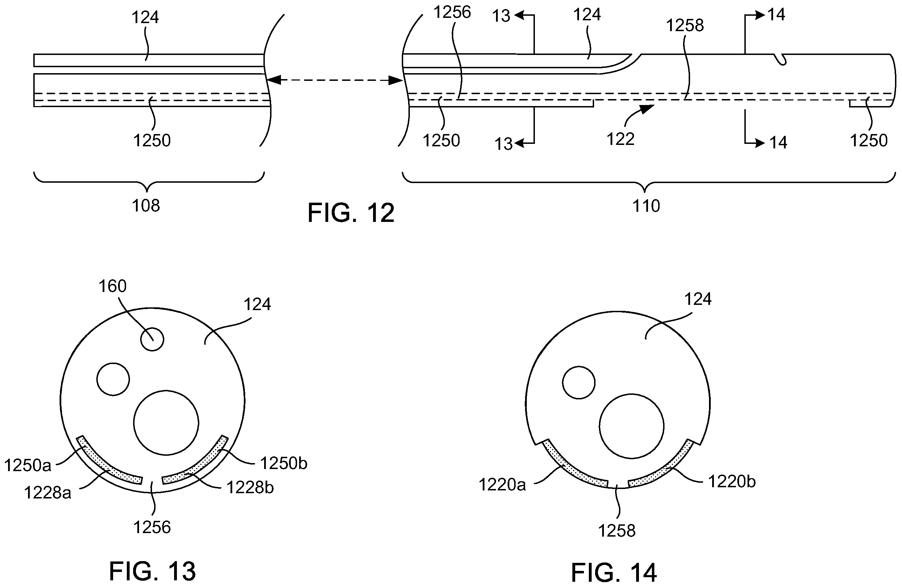

In the example configuration of the tubular member 106 shown in Figs. 9 and 10, the return lumen 950 is a single lumen. Figs. 12-17 show alternative configurations of the tubular member 106, where a plurality of return lumens 1250 may extend in the body 124 from the proximal portion 108 to the distal portion 110. Figs. 12 and 13 show two return lumens 1250a, 1250b, although other numbers of return lumens 1250 greater than two may be disposed in the body 124. Similar to the single return lumen 950 shown in Figs. 9 and 10, each of the return lumens 1250a, 1250b may have an arc-shaped axial cross section and/or together, may circumferentially extend in an arc-shaped manner, as shown in Fig. 13. In addition, as shown in Fig. 13, adjacent return lumens of the plurality of return lumens 1250 may be separated by a divider. For example, in the two return lumen configuration shown in Figs. 12-14, a divider 1256 may separate the two return lumens 1250a, 1250b from each other. The divider 1256 may be formed when the return lumens 1250a, 1250b are extruded in the body 124. When the conductive material making up the unexposed member 128 of the return path is disposed in the return lumens 1250a, 1250b, the divider 1256 may separate the conductive material into a first unexposed member portion 1228a disposed in the first lumen 1250a and a second unexposed member portion 1228b disposed in the second return lumen 1250b.

Also, for some configurations, the divider 1256 may extend in the recess 122, as shown in Figs. 12 and 14. For this configuration, a recess portion 1258 of the divider 1256 disposed in the recess 122 may separate the return electrode 120 into a first return electrode portion 1220a and a second return electrode portion 1220b.

For some example configurations, the body 124, including the divider 1256, may be made of an insulating material, such as polytetrafluoroethylene (PTFE) as an example. When the unexposed member portions 1228a, 1228b are disposed in the respective first and second return lumens 1250a, 1250b, the unexposed member portions 1228a, 1228b may be electrically insulated from each other by the divider 1256. Similarly, the first and second return electrode portions 1220a, 1220b may be electrically insulated from each other by the recess portion 1258 of the divider 1256 extending into the recess 122. In alternative example configurations where the body 124 is not made of an insulating material, or at least does not sufficiently insulate the unexposed member portions 1228a, 1228b and the first and second return electrode portions 1220a, 1220b from each other, the portions 1228a, 1228b, 1220a, 1220b themselves may be coated with an insulating material, such as PTFE for example. For these example configurations, the return path may include two electrically isolated paths-a first return path that includes the first return electrode portion 1220a electrically connected to the first unexposed member portion 1228a, and a second return path that includes the second return electrode portion 1220b electrically connected to the second unexposed member portion 1228b. In operation, each return path may be in electrical communication with a respective output terminal of the output port 144 of the power source 104 (Fig. 1), particularly where the power source 104 is configured to recognize a split neutral electrode.

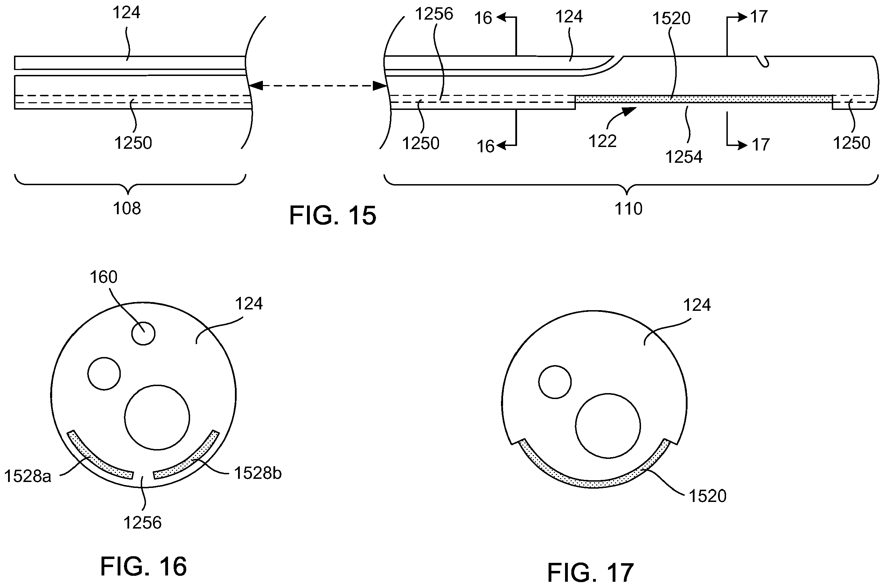

For alternative configurations, as shown in Figs. 15 and 17, the divider 1256 may not extend in the recess 122. For example, after the first and second return lumens 1250a, 1250b are extruded in the body 124 and a spacing or gap 1254 is formed in the body 124, the recess portion 1258 of the divider 1256 (Figs. 12 and 14) may be removed so that the return electrode 120 is a single or continuous piece of conductive material 1520 disposed in the recess 122, as shown in Figs. 15 and 17, while the unexposed member 128 includes a plurality of unexposed member portions 1528a, 1528b disposed in the plurality of return lumens 1250a, 1250b and separated from each other by the divider 1256, as shown in Fig. 16. For configurations where the divider 1256 is made of an electrically insulating material, the exposed member 1528a, 1528b may nonetheless be shorted together by both being electrically connected to the single piece of conductive material making up the return electrode 1520.

In an alternative configuration of the tubular member 106, the conductive material making up the return electrode 120 and the unexposed member 128 of the return path may be embedded in the body 124 of the tubular member 106, as opposed to be inserted and/or disposed in a return lumen. For example, the conductive material may be co-extruded with the body 124. For some example configurations, the embedded conductive material may have an arc-shaped axial cross-section and/or circumferentially extend in an arc-shaped manner, similar to the arc-shaped axial cross-sections shown in Figs. 10, 13, or 14. After co-extruding the conductive material with the body 124, the spacing or gap 906 may be formed, which in turn may expose the portion of the conductive material making of the return electrode 120 to the outer surroundings of the tubular member 106.

The conductive material making up the return electrode 120 and unexposed member 128 of the return path that is disposed in the return lumen 902 or the return lumens 1250, or that is embedded in the body 124, may be one of or combinations of the structures or materials previously described with reference to Figs. 4-8, such as a conductive sheet of metal, film, ink, paste, or wires, as examples. In this way, the return electrode 120 and the unexposed member 128 may be integrated together as parts of the same structure, made of the same material, and/or by having the same configurations. The structure, material, and/or configuration used for the conductive material making up the return electrode 120 and the unexposed member 128 may determine whether one or more return lumens are formed in the body 124 or whether the conductive material is embedded in the body 124. For example, where conductive sheets of metal, ink, film, or paste is used as the conductive material, one or more return lumens may be formed, and the conductive material may be inserted into the one or more return lumens. As another example, where wires are used as the conductive material, the wires may be embedded in and/or co-extruded with the body 124 of the tubular member 106.

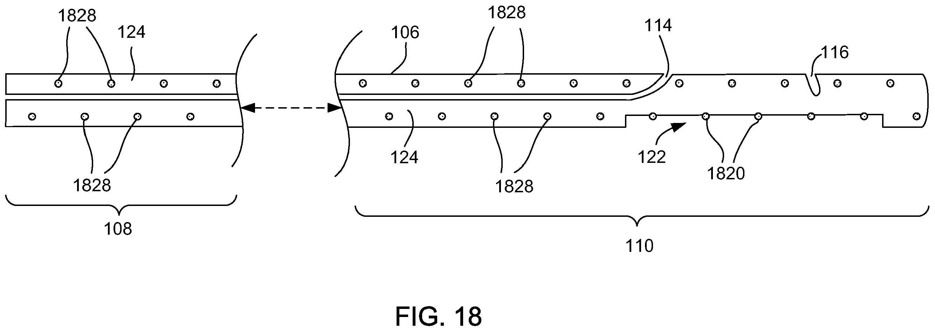

Fig. 18 shows a cross-sectional side view of an example configuration where the return electrode and the unexposed member are integrated together as parts of a coil or a coil-like structure embedded in the body 124 of the tubular member 106. The coil may be co-extruded with the body 124. After the co-extrusion process, a spacing or gap 1854 of the body 124 may be removed from the body 124, exposing portions of some of the coil segments. The exposed portions of the coil segments 1820 may make up the return electrode, while the remaining portions of the coil segments 1828 that are still embedded may make up the unexposed member of the return path. For some configurations, the exposed portions 1820 may correspond to the plurality of angled wires 820 of the wired configuration shown in Fig. 8.

The example configurations in Figs. 9-18 show midpoints of the various configurations of the return electrode, the unexposed member, and/or the one or more return lumens circumferentially disposed opposite to and/or 180-degrees from a cutting wire lumen 160 configured to have the cutting wire 112 moveably disposed therein (as shown in Figs. 10, 11A, 13 and 16). This circumferential positioning is similar to the 180-degree positioning of the recess 122 and return electrode 120 shown in Fig. 2. Alternatively, the midpoints of the various configurations of the return electrode, the unexposed member, and/or the one or more return lumens shown in Figs. 9-18 may be circumferentially positioned a predetermined number of degrees 0 from the 180-degree position, similar to the configuration shown in Fig. 3.

Additionally, the example configurations in Figs. 9-18 show the various configurations of the unexposed member of the return path and/or the one or more return lumens proximally extending to the proximal portion 108 of the tubular member 106. Alternatively, the unexposed member of the return path and/or the one or more return lumens may not proximally extend to the proximal portion 108. For example, the unexposed member and/or the one or more return lumens may only be disposed at the distal portion 110. In addition or alternatively, at least some of the unexposed member may not be integrated together with the return electrode as parts of the same structure or made of the same material. For these alternative configurations, at least a proximal part of the unexposed member 128 may be a return wire or other conductive member disposed within or otherwise integrated with the proximal portion 108 of the tubular member 106. A distal end of the return wire may be coupled with a proximal end of the return electrode or a distal part of the unexposed member that is integral with the return electrode so that the power source 104 (Fig. 1) is electrically coupled to the return electrode 120. As an example, the tubular member 106 may be extruded with a return lumen extending from the proximal portion 108 to the distal portion 110. A recess 122 may be formed in the distal portion 110, as previously described. The unexposed member 128 may include a return wire extending in the return lumen from the proximal portion 108 to the recess 122. The recess 122 may be filled with a conductive material making up a return electrode 120 (or the conductive material is otherwise inserted into the recess 122). The recess 122 may be filled such that conductive material contacts and/or is connected to a distal end of the return wire.

## Claims

1. A bipolar sphincterotome (102) configured to perform an electrosurgical procedure at a treatment site within a patient, the sphincterotome comprising:
an elongate tubular member (106) longitudinally extending from a proximal portion (108) to a distal portion (110), the tubular member comprising:
a body (106);
a cutting wire lumen longitudinally extending in the body; and
a recess (122) extending in the body at the distal portion;
an active path configured for delivery of electrical current generated from a power source to the distal portion, wherein the active path comprises an exposed active portion disposed outside of the tubular member to contact tissue at the treatment site; and
a return path for return of the electrical current from the distal portion to the power source, wherein the return path comprises a return electrode (120) disposed in the recess at the distal portion;
wherein the active path comprises a cutting wire movably disposed and longitudinally extending in the cutting wire lumen, and
wherein the exposed active portion comprises a cutting edge (118) disposed outside of and longitudinally extending along the body of the tubular member at the distal portion wherein
the tubular member has a central longitudinal axis;
the cutting edge is distally secured to the body of the tubular member at an anchor point (116), said anchor point being positioned at a first circumferential position about the central longitudinal axis;
a midpoint (206) of the return electrode is disposed at a second circumferential position about the central longitudinal axis, said second circumferential position being offset from a circumferential position that is 180-degrees from said first circumferential position by a predetermined non-zero number of degrees (θ) about the central longitudinal axis.

2. The bipolar sphincterotome of claim 1, wherein each of the recess and the return electrode at least one of:
proximally extends past a proximal opening in the body where the cutting edge exits the body; or
distally extends past an anchor point where the cutting edge is distally secured to the body.

3. The bipolar sphincterotome of claim 1, wherein the midpoint of the return electrode is circumferentially disposed at an offset from a position that is 180-degree from a circumferential position of the exposed active portion.

4. The bipolar sphincterotome according to any of claims 1 to 3, wherein the return electrode comprises conductive ink or conductive film.

5. The bipolar sphincterotome according to any of claims 1 to 3, wherein the return electrode comprises one or more conductive wires.

6. The bipolar sphincterotome of claim 5, wherein the one or more conductive wires are made of nitinol.

7. The bipolar sphincterotome according to claims 5 or 6, wherein the one or more conductive wires comprises a plurality of wires having a mesh configuration or a braided configuration.

8. The bipolar sphincterotome of claim 1, wherein the return path further comprises a coil, wherein the return electrode comprises an exposed portion of the coil, and wherein a remaining portion of the coil is embedded in the body of the tubular member.

9. The bipolar sphincterotome according to any of claims 1 to 8, wherein the tubular member further comprises a return lumen longitudinally extending in the distal portion, wherein the recess comprises a recess portion of the return lumen, the recess further comprising a gap extending from an outer surface of the tubular member to the recess portion of the return lumen.

10. The bipolar sphincterotome of claim 9, wherein the return lumen has an arc-shaped axial cross-section.

11. The bipolar sphincterotome according to claim 9 or 10, wherein the return path further comprises a conductive, unexposed return member connected to the return electrode, the unexposed return member disposed in the return lumen proximal the return electrode.

12. The bipolar sphincterotome of claim 11, wherein the return electrode and the unexposed return member are integrated together as parts of a same structure or a same conductive material.

13. The bipolar sphincterotome of claim 11, wherein the return lumen comprises a first return lumen portion and a second return lumen portion that are separated from each other by a divider, and
wherein the unexposed return member comprises a first unexposed portion and a second unexposed portion, the first unexposed portion disposed in the first return lumen portion and the second unexposed portion disposed in the second return lumen portion.

14. The bipolar sphincterotome of claim 13, wherein the return electrode comprises a single piece of conductive material, and wherein the first unexposed portion and the second unexposed portion are each electrically connected to the single piece of conductive material.

15. The bipolar sphincterotome of claim 13, wherein the divider longitudinally extends in the recess,
wherein the divider is made of an electrically insulating material,
wherein the return electrode comprises a first return electrode portion and a second return electrode portion separated and electrically insulated from each other by the divider,
wherein the return path comprises:
a first return path portion comprising the first unexposed portion electrically connected to the first return electrode portion; and
a second return path portion comprising the second unexposed portion electrically connected to the second return electrode portion,
wherein the first and second return path portions are electrically insulated from each other by the divider.

## Patentansprüche

1. Bipolares Sphinkterotom (102), gestaltet zum Durchführen eines elektrochirurgischen Verfahrens an einer Behandlungsstelle in einem Patienten, wobei das Sphinkterotom umfasst:
ein langgestrecktes rohrförmiges Element (106), das in Längsrichtung von einem proximalen Teil (108) zu einem distalen Teil (110) verläuft, wobei das rohrförmige Element umfasst:
einen Körper (106);
ein Schneiddrahtlumen, das in Längsrichtung in dem Körper verläuft; und
eine Vertiefung (122), die an dem distalen Teil in dem Körper verläuft;
eine Wirkbahn, die dafür gestaltet ist, von einer Energiequelle erzeugten elektrischen Strom zu dem distalen Teil zu führen, wobei die Wirkbahn einen freiliegenden aktiven Teil umfasst, der außerhalb des rohrförmigen Elements angeordnet ist, um mit Gewebe an der Behandlungsstelle in Kontakt zu kommen; und
eine Rückleitung zum Rückleiten des elektrischen Stroms von dem distalen Teil zu der Stromquelle, wobei die Rückleitung eine Rückleitungselektrode (120) umfasst, die in der Vertiefung an dem distalen Teil angeordnet ist;
wobei die Wirkbahn einen Schneiddraht umfasst, der beweglich in dem Schneiddrahtlumen angeordnet ist und in Längsrichtung darin verläuft; und
wobei der freiliegende aktive Teil eine Schneidkante (118) umfasst, die an dem distalen Teil außerhalb des Körpers des rohrförmigen Elements angeordnet ist und in Längsrichtung daran entlang verläuft;
wobei das rohrförmige Element eine zentrale Längsachse aufweist;
die Schneidkante an dem Körper des rohrförmigen Elements distal an einem Ankerpunkt (116) befestigt ist, wobei der Ankerpunkt an einer ersten Umfangsstellung um die zentrale Längsachse angeordnet ist;
der Mittelpunkt (206) der Rückleitungselektrode an einer zweiten Umfangsstellung um die zentrale Längsachse angeordnet ist, wobei die zweite Umfangsstellung bezogen auf eine Umfangsstellung, die 180 Grad von der ersten Umfangsstellung entfernt ist, um eine vorbestimmte, von null verschiedene Zahl von Graden (θ) um die zentrale Achse versetzt ist.

2. Bipolares Sphinkterotom gemäß Anspruch 1, wobei jedes von der Vertiefung und der Rückleitungselektrode wenigstens eines von:
proximal über eine proximale Öffnung in dem Körper, an der die Schneidkante aus dem Körper austritt, hinaus verläuft; oder
distal über einen Ankerpunkt, an dem die Schneidkante distal an dem Körper befestigt ist, hinaus verläuft.

3. Bipolares Sphinkterotom gemäß Anspruch 1, wobei der Mittelpunkt der Rückleitungselektrode in Umfangsrichtung mit einer Versetzung von einer Stellung angeordnet ist, die 180 Grad von der Umfangsstellung des freiliegenden aktiven Teils angeordnet ist.

4. Bipolares Sphinkterotom gemäß einem der Ansprüche 1 bis 3, wobei die Rückleitungselektrode leitfähige Tinte oder einen leitfähigen Film umfasst.

5. Bipolares Sphinkterotom gemäß einem der Ansprüche 1 bis 3, wobei die Rückleitungselektrode einen oder mehrere leitfähige Drähte umfasst.

6. Bipolares Sphinkterotom gemäß Anspruch 5, wobei der eine oder die mehreren leitfähigen Drähte aus Nitinol bestehen.

7. Bipolares Sphinkterotom gemäß Anspruch 5 oder 6, wobei der eine oder die mehreren leitfähigen Drähte eine Vielzahl von Drähten umfassen, die eine Netzkonfiguration oder eine geflochtene Konfiguration aufweisen.

8. Bipolares Sphinkterotom gemäß Anspruch 1, wobei die Rückleitung ferner eine Spule umfasst, wobei die Rückleitungselektrode einen freiliegenden Teil der Spule umfasst und wobei ein restlicher Teil der Spule in dem Körper des rohrförmigen Elements eingebettet ist.

9. Bipolares Sphinkterotom gemäß einem der Ansprüche 1 bis 8, wobei das rohrförmige Element ferner ein Rückleitungslumen umfasst, das in Längsrichtung in dem distalen Teil verläuft, wobei die Vertiefung einen Vertiefungsteil des Rückleitungslumens umfasst, wobei die Vertiefung ferner einen Spalt umfasst, der von einer Außenfläche des rohrförmigen Elements zu dem Vertiefungsteil des Rückleitungslumens verläuft.

10. Bipolares Sphinkterotom gemäß Anspruch 9, wobei das Rückleitungslumen einen bogenförmigen axialen Querschnitt aufweist.

11. Bipolares Sphinkterotom gemäß Anspruch 9 oder 10, wobei die Rückleitung ferner ein leitfähiges, nicht freiliegendes Rückleitungselement umfasst, das mit der Rückleitungselektrode verbunden ist, wobei das nicht freiliegende Rückleitungselement in dem Rückleitungslumen proximal bezogen auf die Rückleitungselektrode angeordnet ist.

12. Bipolares Sphinkterotom gemäß Anspruch 11, wobei die Rückleitungselektrode und das nicht freiliegendes Rückleitungselement zusammen als Teile der gleichen Struktur oder als das gleiche leitfähige Material integriert sind.

13. Bipolares Sphinkterotom gemäß Anspruch 11, wobei das Rückleitungslumen einen ersten Rückleitungslumenteil und einen zweiten Rückleitungslumenteil umfasst, die durch ein Trennelement voneinander getrennt sind, und
wobei das nicht freiliegende Rückleitungselement einen ersten nicht freiliegenden Teil und einen zweiten nicht freiliegenden Teil umfasst, wobei der erste nicht freiliegende Teil in dem ersten Rückleitungslumenteil angeordnet ist und der zweite nicht freiliegende Teil in dem zweiten Rückleitungslumenteil angeordnet ist.

14. Bipolares Sphinkterotom gemäß Anspruch 13, wobei die Rückleitungselektrode ein einziges Stück von leitfähigem Material umfasst und wobei der erste nicht freiliegende Teil und der zweite nicht freiliegende Teil jeweils elektrisch mit dem einzigen Stück von leitfähigem Material verbunden sind.

15. Bipolares Sphinkterotom gemäß Anspruch 13, wobei das Trennelement in Längsrichtung in der Vertiefung verläuft,
wobei das Trennelement aus einem elektrisch isolierenden Material besteht,
wobei die Rückleitungselektrode einen ersten Rückleitungselektrodenteil und einen zweiten Rückleitungselektrodenteil umfasst, die durch das Trennelement getrennt und elektrisch voneinander isoliert sind,
wobei die Rückleitung umfasst:
einen ersten Rückleitungsteil, umfassend den ersten nicht freiliegenden Teil, der elektrisch mit dem ersten Rückleitungselektrodenteil verbunden ist; und
einen zweiten Rückleitungsteil, umfassend den zweiten nicht freiliegenden Teil, der elektrisch mit dem ersten Rückleitungselektrodenteil verbunden ist,
wobei der erste und der zweite Rückleitungsteil durch das Trennelement elektrisch voneinander isoliert sind.

## Revendications

1. Sphinctérotome bipolaire (102) conçu pour exécuter une intervention électrochirurgicale au niveau d'un site de traitement dans un patient, le sphinctérotome comprenant :
un élément tubulaire allongé (106) s'étendant longitudinalement depuis une partie proximale (108) vers une partie distale (110), l'élément tubulaire comprenant :
un corps (106) ;
une lumière de fil coupant s'étendant longitudinalement dans le corps ; et
un renfoncement (122) s'étendant dans le corps au niveau de la partie distale ;
un trajet actif conçu pour l'administration d'un courant électrique produit par une source d'énergie à une partie distale, le trajet actif comprenant une partie active apparente disposée en dehors de l'élément tubulaire pour entrer en contact avec un tissu au niveau du site de traitement ; et
un trajet de retour pour le retour du courant électrique depuis la partie distale vers la source d'énergie, le trajet de retour comprenant une électrode de retour (120) disposée dans le renfoncement au niveau de la partie distale ;
dans lequel le trajet actif comprend un fil coupant disposé mobile et s'étendant longitudinalement dans la lumière de fil coupant, et
dans lequel la partie active apparente comprend un bord coupant (118) disposé en dehors du corps de l'élément tubulaire et s'étendant longitudinalement depuis celui-ci au niveau de la partie distale
dans lequel
l'élément tubulaire a un axe longitudinal central ;
le bord coupant est fixé distalement au corps de l'élément tubulaire au niveau d'un point d'ancrage (116), ledit point d'ancrage étant positionné au niveau d'une première position circonférentielle autour de l'axe longitudinal central ;
un point médian (206) de l'électrode de retour est disposé au niveau d'une seconde position circonférentielle autour de l'axe longitudinal central, ladite seconde position circonférentielle étant décalée d'une position circonférentielle qui est à 180 degrés de ladite première position circonférentielle à hauteur d'un nombre de degrés non nul prédéfini (θ) autour de l'axe longitudinal central.

2. Sphinctérotome bipolaire selon la revendication 1,
dans lequel chacun du renfoncement et de l'électrode de retour :
s'étend proximalement au-delà d'une ouverture proximale dans le corps là où le bord coupant sort du corps ; et/ou
s'étend distalement au-delà d'un point d'ancrage là où le bord coupant est fixé distalement au corps.

3. Sphinctérotome bipolaire selon la revendication 1, dans lequel le point médian de l'électrode de retour est disposé circonférentiellement au niveau d'un décalage depuis une position qui est à 180 degrés d'une position circonférentielle de la partie active apparente.

4. Sphinctérotome bipolaire selon l'une quelconque des revendications 1 à 3, dans lequel l'électrode de retour comprend une encre conductrice ou un film conducteur.

5. Sphinctérotome bipolaire selon l'une quelconque des revendications 1 à 3, dans lequel l'électrode de retour comprend un ou plusieurs fils conducteurs.

6. Sphinctérotome bipolaire selon la revendication 5, dans lequel le ou les fils conducteurs sont en nitinol.

7. Sphinctérotome bipolaire selon la revendication 5 ou 6, dans lequel le ou les fils conducteurs comprennent une pluralité de fils ayant une configuration maillée ou une configuration tressée.

8. Sphinctérotome bipolaire selon la revendication 1, dans lequel le trajet de retour comprend en outre une bobine, dans lequel l'électrode de retour comprend une partie apparente de la bobine, et dans lequel une partie restante de la bobine est intégrée dans le corps de l'élément tubulaire.

9. Sphinctérotome bipolaire selon l'une quelconque des revendications 1 à 8, dans lequel l'élément tubulaire comprend en outre une lumière de retour s'étendant longitudinalement dans la partie distale, dans lequel le renfoncement comprend une partie renfoncement de la lumière de retour, le renfoncement comprenant en outre un espace s'étendant depuis une surface externe de l'élément tubulaire vers la partie renfoncement de la lumière de retour.

10. Sphinctérotome bipolaire selon la revendication 9, dans lequel la lumière de retour a une coupe transversale axiale en forme d'arc.

11. Sphinctérotome bipolaire selon la revendication 9 ou 10, dans lequel le trajet de retour comprend en outre un élément de retour conducteur non apparent connecté à l'électrode de retour, l'élément de retour non apparent étant disposé dans la lumière de retour à proximité de l'électrode de retour.

12. Sphinctérotome bipolaire selon la revendication 11, dans lequel l'électrode de retour et l'élément de retour non apparent sont intégrés ensemble en tant que parties d'une même structure ou d'un même matériau conducteur.

13. Sphinctérotome bipolaire selon la revendication 11, dans lequel la lumière de retour comprend une première partie lumière de retour et une seconde partie lumière de retour qui sont séparées l'une de l'autre par un élément de division, et
dans lequel l'élément de retour non apparent comprend une première partie non apparente et une seconde partie non apparente, la première partie non apparente étant disposée dans la première partie lumière de retour et la seconde partie non apparente étant disposée dans la seconde partie lumière de retour.

14. Sphinctérotome bipolaire selon la revendication 13, dans lequel l'électrode de retour comprend une pièce unique de matériau conducteur, et dans lequel la première partie non apparente et la seconde partie non apparente sont chacune connectées électriquement à la pièce unique de matériau conducteur.

15. Sphinctérotome bipolaire selon la revendication 13, dans lequel l'élément de division s'étend longitudinalement dans le renfoncement,
dans lequel l'élément de division est en matériau électriquement isolant,
dans lequel l'électrode de retour comprend une première partie électrode de retour et une seconde partie électrode de retour séparées et électriquement isolées l'une de l'autre par l'élément de division,
dans lequel le trajet de retour comprend :
une première partie trajet de retour comprenant la première partie non apparente électriquement connectée à la première partie électrode de retour ; et
une seconde partie trajet de retour comprenant la seconde partie non apparente électriquement connectée à la seconde partie électrode de retour,
dans lequel les première et seconde parties trajet de retour sont électriquement isolées l'une de l'autre par l'élément de division.
